(19) Europäisches Patentamt
European Patent Office
Office européen des brevets

(11) Veröffentlichungsnummer: **0 197 322**
**A2**

# (12) EUROPÄISCHE PATENTANMELDUNG

(21) Anmeldenummer: **86103102.9**

(22) Anmeldetag: **08.03.86**

(51) Int. Cl.[4]: **C07D 249/08**, A01N 43/653

(30) Priorität: **22.03.85 DE 3510411**

(43) Veröffentlichungstag der Anmeldung: **15.10.86 Patentblatt 86/42**

(84) Benannte Vertragsstaaten: **AT BE CH DE FR GB IT LI NL SE**

(71) Anmelder: **BAYER AG**
**Konzernverwaltung RP Patentabteilung**
**D-5090 Leverkusen 1 Bayerwerk(DE)**

(72) Erfinder: **Holmwood, Graham, Dr.**
**Krutscheider Weg 105**
**D-5600 Wuppertal 1(DE)**
Erfinder: **Büchel, Karl Heinz, Prof. Dr.**
**Dabringhausener Strasse 42**
**D-5093 Burscheid(DE)**
Erfinder: **Reinecke, Paul, Dr.**
**Steinstrasse 8**
**D-5090 Leverkusen 3(DE)**
Erfinder: **Brandes, Wilhelm, Dr.**
**Eichendorffstrasse 3**
**D-5653 Leichlingen(DE)**
Erfinder: **Hänssler, Gerd, Dr.**
**Am Arenzberg 58a**
**D-5090 Leverkusen(DE)**

(54) **Triazol-Derivate.**

(57) Neue Triazol-Derivate der Formel

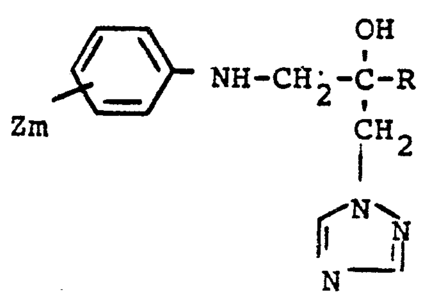

(I)

in welcher

R für Alkyl, Cycloalkyl oder für die Gruppierungen

EP 0 197 322 A2

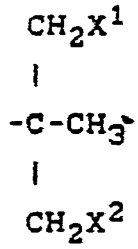 oder 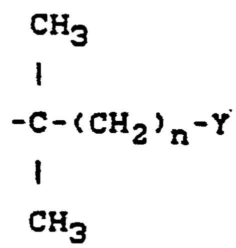 steht,

worin

X¹ für Wasserstoff oder Halogen steht,

X² für Halogen steht,

Y für Alkoxy, Alkylthio, Halogenalkoxy, Halogenalkylthio, Alkenyl, Alkoxycarbonyl, Cyano, gegebenenfalls substituiertes Phenyl, gegebenenfalls substiutiertes Phenoxy, gegebenenfalls substituiertes Phenylthio, gegebenenfalls substituiertes Phenylalkoxy oder gegebenenfalls substituiertes Phenylalkyl steht und

n für die Zahlen 0, 1 oder 2 steht,

Z für Halogen, Alkyl, Halogenalkyl, Halogenalkoxy, Halogenalkylthio oder Phenyl steht und

m für die Zahlen 0, 1, 2 oder 3 steht,

sowie deren Säureadditions-Salze und Metallsalz-Komplexe,

mehrere Verfahren zur Herstellung der neuen Stoffe und deren Verwendung als Fungizide und Pflanzenwachstumsregulatoren.

# Triazol-Derivate

Die vorliegende Erfindung betrifft neue Triazol-Derivate, mehrere Verfahren zu ihrer Herstellung und ihre Verwendung als Fungizide und Pflanzenwachstumsregulatoren.

Es ist bereits bekannt geworden, daß zahlreiche 1-Hydroxyalkyl-azolyl-Derivate fungizide und pflanzenwachstumsregulierende Eigenschaften besitzen (vgl. DE-OS 3 202 601). So läßt sich z.B. 2-(4-Chlorphenylthiomethyl)-3,3-dimethyl-1-(1,2,4-triazol-1-yl)-butan-2-ol als Fungizid verwenden. Die Wirksamkeit dieses Stoffes ist jedoch vor allem bei niedrigen Aufwandmengen nicht immer befriedigend.

Ferner ist bereits bekannt geworden daß Zinkethylen-1.2-bisdithiocarbamidat gut zur Bekämpfung von pilzlichen Pflanzenkrankheiten eingesetzt werden kann (vgl. Phytopathology 33, 1113 (1963)). Die Verwendung dieses Stoffes ist aber dadurch beeinträchtigt, daß die Wirkung bei niedrigen Dosierungen nicht immer ausreichend ist.

Es wurden nun neue Triazol-Derivate der Formel

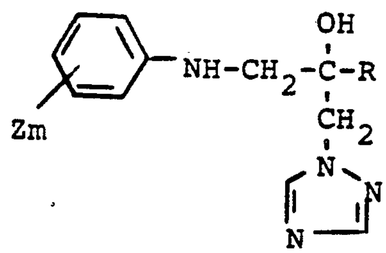

(I)

in welcher

R für Alkyl, Cycloalkyl oder für die Gruppierungen

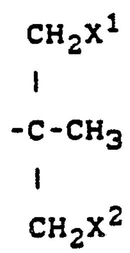

oder

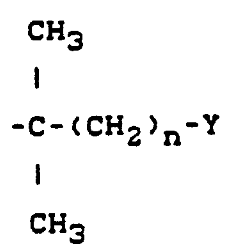

steht,

worin

$X^1$ für Wasserstoff oder Halogen steht,

$X^2$ für Halogen steht,

Y für Alkoxy, Alkylthio, Halogenalkoxy, Halogenaklylthio, Alkenyl, Alkoxycarbonyl, Cyano, gegebenenfalls substituiertes Phenyl, gegebenenfalls substituiertes Phenoxy, gegebenenfalls substituiertes Phenylthio, gegebenenfalls substituiertes Phenylalkoxy oder gegebenenfalls substituiertes Phenylalkylthio steht und

n für die Zahlen 0, 1 oder 2 steht

Z für Halogen, Alkyl, Halogenalkyl, Halogenalkoxy, Halogenalkylthio oder Phenyl steht und

m für die Zahlen 0, 1, 2 oder 3 steht,

sowie deren Säureadditions-Salze und Metallsalz-Komplexe gefunden.

Die Verbindungen der Formel (I) besitzen ein asymmetrisch substituiertes Kohlenstoffatom und können deshalb in den beiden optischen Isomerenformen anfallen. Die vorliegende Erfindung betrifft sowohl die Isomerengemische als auch die einzelnen Isomeren.

Weiterhin wurde gefunden, daß man neue Triazol-Derivate der Formel (I) sowie deren Säureadditions-Salze und Metallsalz-Komplexe erhält, wenn man

a) Oxirane der Formel

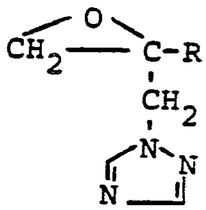

(II)

in welcher

R die oben angegebene Bedeutung hat,

mit Anilin-Derivaten der Formel

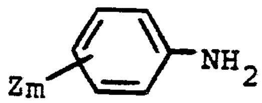

(III)

in welcher

Z und m die oben angegebene Bedeutung haben,

in Gegenwart eines Verdünnungsmittels umsetzt; oder

b) Acetanilide der Formel

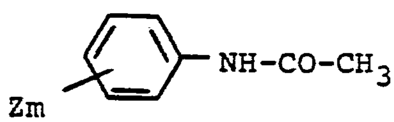

(IV)

in welcher

Z und m die oben angegebene Bedeutung haben,

mit starken Basen in Gegenwart eines Verdünnungsmittels behandelt und die dabei entstehenden Produkte anschließend mit Oxiranen der Formel

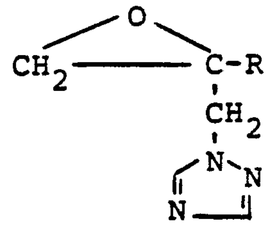

(II)

in welcher

R die oben angegebene Bedeutung hat,

in Gegenwart eines Verdünnungsmittels umsetzt;

und gegebenenfalls anschließend an die erhaltenen Verbindungen der Formel (I) eine Säure oder ein Metallsalz addiert.

Außerdem wurde gefunden, daß die erfindungsgemäßen Stoffe starke fungizide und pflanzenwuchsregulierende Eigenschaften besitzen.

Überraschenderweise zeigen die erfindungsgemäßen Wirkstoffe der Formel (I) sowie deren Säureadditions-Salze und Metallsalz-Komplexe eine deutlich bessere fungizide und pflanzenwuchsregulierende Wirkung als das 2-(4-Chlorphenyl-thiomethyl)-3,3-dimethyl-1-(1,2,4-triazol-1-yl)-butan-2-ol, welches ein konstitutionell ähnlicher vorbekannter Wirkstoff gleicher Indikation ist. Außerdem besitzen die erfindungsgemäßen Verbindungen auch eine bessere fungizide Wirkung als das aus dem Stand der Technik vorbekannte Zinkethylen-1,2-bis-dithiocarbamidat, welches eine wirkungsmäßig naheliegende Verbindung ist.

Die erfindungsgemäßen Triazol-Derivate sind durch die Formel (I) allgemein definiert. In dieser Formel steht R vorzugsweise für geradkettiges oder verzweigtes Alkyl mit 1 bis 6 Kohlenstoffatomen, Cycloalkyl mit 3 bis 8 Kohlenstoffatomen oder für die Gruppierungen der Formeln

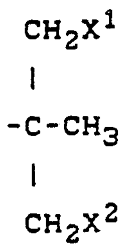 und 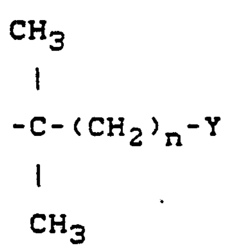 worin

X' vorzugsweise für Wasserstoff, Fluor, Chlor oder Brom steht,

X² vorzugsweise für Fluor, Chlor oder Brom steht,

Y vorzugsweise für Alkoxy mit 1 bis 6 Kohlenstoffatomen, Alkylthio mit 1 bis 6 Kohlenstoffatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkenyl mit 2 bis 6 Kohlenstoffatomen, Alkoxycarbonyl mit 1 bis 4 Kohlenstoffatomen im Alkoxyteil, Cyano, Phenyl, Phenoxy, Phenylalkoxy mit 1 bis 4 Kohlenstoffatomen im Alkoxyteil und Phenylalkylthio mit 1 bis 4 Kohlenstoffatomen im Alkylthioteil steht, wobei jeder dieser Phenyl-, Phenoxy-, Phenylalkoxy-und Phenylalkylthio-Reste ein-oder mehrfach, gleichartig oder verschieden substituiert sein kann durch Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleiche oder verschiedenen Halogenatomen, Cyclohexyl, Dialkylamino mit 1 bis 4 Kohlenstoffatomen in jedem Alkylteil, Nitro, Cyano und Alkoxycarbonyl mit 1 bis 4 Kohlenstoffatomen im Alkoxyteil,

und

n für die Zahlen 0, 1 oder 2 steht,

Z steht vorzugsweise für Fluor, Chlor, Brom, Alkyl

mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen oder für Phenyl und

m steht für die Zahlen 0, 1 oder 3.

Besonders bevorzugt sind diejenigen Verbindungen der Formel (I), in denen

R für geradkettiges oder verzweigtes Alkyl mit 1 bis 4 Kohlenstoffatomen, Cycloalkyl mit 3 bis 6 Kohlenstoffatomen oder für die Gruppierungen der Formeln

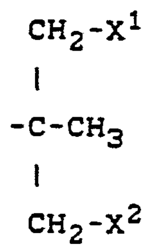

und

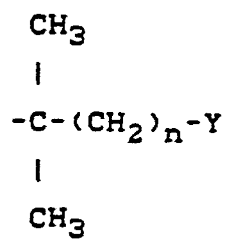

steht, worin

$X^1$ für Wasserstoff, Fluor, Chlor oder Brom steht,

$X^2$ für Fluor, Chlor oder Brom steht,

Y für Methoxy, Ethoxy, Methylthio, Ethylthio, Trifluormethyl, Trifluormethylthio, Vinyl, Methoxycarbonyl, Ethoxycarbonyl, Cyano sowie für Phenyl, Phenoxy, Phenylmethoxy und Phenylmethylthio steht, wobei jeder dieser Phenyl-, Phenoxy-, Phenylmethoxy-und Phenylmethylthio-Reste im Phenylteil ein bis dreifach, gleichartig oder verschieden substituiert sein kann durch Fluor, Chlor, Methyl, Ethyl, Methoxy, Methylthio, Trifluormethyl, Trifluormethoxy, Trifluormethylthio, Cyclohexyl, Dimethylamino, Methoxycarbonyl und/oder Ethoxycarbonyl,

n für die Zahlen 0, 1 oder 2 steht,

Z für Fluor, Chlor, Brom, Methyl, Ethyl, tert.-Butyl, Trifluormethyl, Trifluormethoxy, Trifluormethylthio oder Phenyl steht und

m für die Zahlen 0, 1, 2 oder 3 steht.

Bevorzugte erfindungsgemäße Verbindungen sind auch Additionsprodukte aus Säuren und denjenigen Triazol-Derivaten der Formel (I), in denen die Substituenten R und Z sowie der Index m die Bedeutungen haben, die bereits vorzugsweise genannt wurden.

Zu den Säuren die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono-und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt-und der I. und II. sowie IV. bis VIII. Nebengruppe des Periodensystems der Elemente und denjenigen Triazol-Derivaten der Formel (I), in denen die Substituenten R und Z sowie der Index m die Bedeutungen haben, die bereits vorzugsweise genannt werden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man 2-tert.-Butyl-2-(1,2,4-triazol-1-yl-methyl)-oxiran und 3.4-Dichloranilin als Ausgangsstoffe, so kann der Verlauf des erfindungsgemäßen Verfahrens (a) durch das folgende Formelschema wiedergegeben werden:

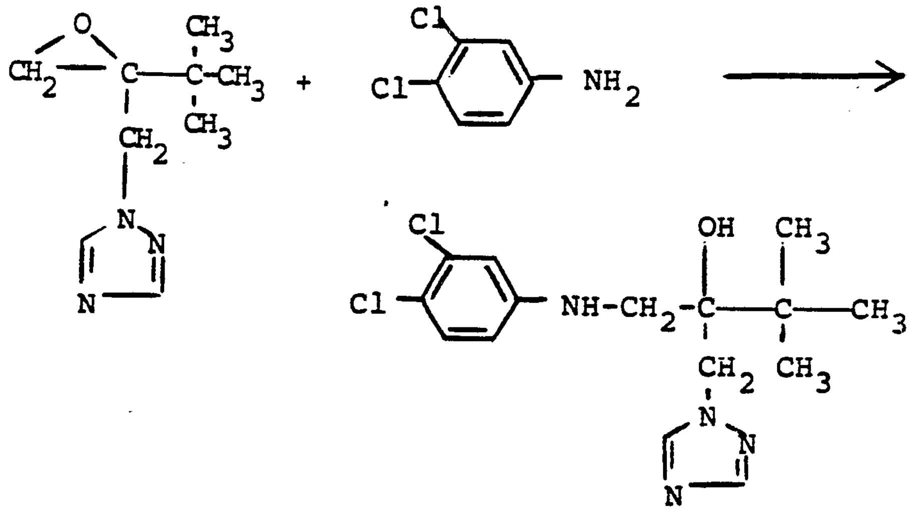

Verwendet man 4-Chlor-acetanilid als Ausgangsstoff, Natriumhydrid als Base und 2-tert.-Butyl-2-(1,2,4-triazol-1-yl-methyl)-oxiran als Reaktionskomponente, so kann der Verlauf des erfindungsgemäßen Verfahrens (b) durch das folgende Formelschema wiedergegeben werden:

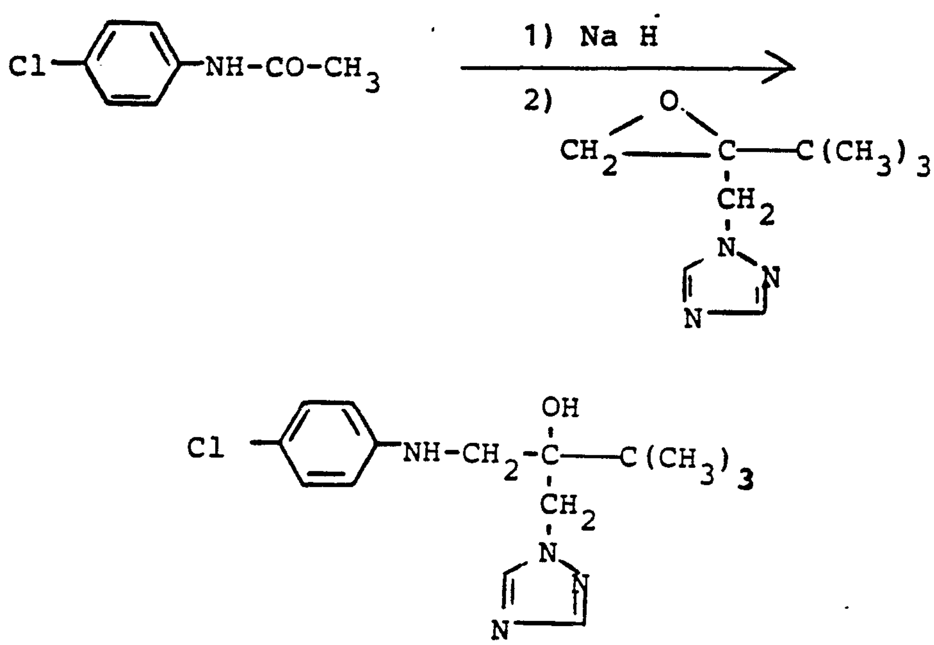


Die bei der Durchführung des erfindungsgemäßen Verfahrens (a) als Ausgangsstoffe benötigten Oxirane sind durch die Formel (II) definiert.

In dieser Formel hat R vorzugsweise diejenigen Bedeutungen, die bereits im Zusammenhang mit der Beschreibung der erfindungsgemäßen Stoffe der Formel (I) vorzugsweise für diesen Substituenten genannt wurden.

Die Oxirane der Formel (II) sind bereits bekannt oder lassen sich nach im Prinzip bekannten Methoden in einfacher Weise herstellen (vgl. DE-OS 3 202 601).

Die bei der Durchführung des erfindungsgemäßen Verfahrens (a) weiterhin als Ausgangsstoffe benötigten Anilin-Derivate sind durch die Formel (III) definiert. In dieser Formel haben Z und m vorzugsweise diejenigen Bedeutungen, die bereits im Zusammenhang mit der Beschreibung der erfindungsgemäßen Stoffe der Formel (I) vorzugsweise für diesen Substituenten bzw. diesen Index genannt wurden.

Die Anilin-Derivate der Formel (III) sind allgemein bekannte Verbindungen.

Als Verdünnungsmittel kommen bei der Umsetzung des erfindungsgemäßen Verfahrens (a) alle polaren inerten Solventien in Frage. Vorzugsweise verwendbar sind Alkohole, wie Methanol, Ethanol und Propanol, sowie stark polare organische Solventien, wie Dimethylformamid und Dimethylsulfoxid, und außerdem Wasser.

Besonders bevorzugt sind Gemische aus polaren organischen Lösungsmitteln und Wasser, z.B. Gemische aus Alkoholen, wie Ethanol, und Wasser.

Die Reaktionstemperaturen können bei der Durchführung des erfindungsgemäßen Verfahrens - (a) innerhalb eines größeren Bereiches variiert werden. Im allgemeinen arbeitet man bei Temperaturen zwischen 20 und 180°C, vorzugsweise zwischen 60 und 150°C.

Das erfindungsgemäße Verfahren (a) wird im allgemeinen unter Normaldruck durchgeführt. Es ist jedoch auch möglich, unter einem erhöhten Druck von bis zu 10 bar oder unter vermindertem Druck zu arbeiten.

Bei der Durchführung des erfindungsgemäßen Verfahrens (a) geht man im allgemeinen so vor, daß man auf 1 Mol an Oxiran der Formel (II) 1 bis 5 Mol, vorzugsweise 1 bis 3 Mol an Anilin-Derivat der Formel (III) einsetzt. Die Aufarbeitung erfolgt nach üblichen Methoden. Im allgemeinen geht man so vor, daß man das Reaktionsgemisch einengt, den Rückstand mit Wasser versetzt, das dabei entstehende Gemisch mit einem organischen Lösungsmittel extrahiert, die organische Phase einengt und den verbleibenden Rückstand chromatographisch bzw. durch Umkristallisation reinigt.

Die bei dem erfindungsgemäßen Verfahren (b) als Ausgangsstoffe benötigten Acetanilide sind durch die Formel (IV) definiert. In dieser Formel haben Z und m vorzugs weise diejenigen Bedeutungen, die bereits im Zusammenhang mit der Beschreibung der erfindungsgemäßen Stoffe der Formel (I) vorzugsweise für diesen Substituenten bzw. diesen Index genannt wurden.

Die Acetanilide der Formel (IV) sind allgemein bekannte Stoffe der organischen Chemie.

Als starke Basen kommen bei der Durchführung des erfindungsgemäßen Verfahrens - (b) alle üblichen starken anorganischen und organischen Basen in Frage. Vorzugsweise verwendbar sind Alkalihydride, wie Natriumhydrid, ferner Lithium-Verbindungen, wie Butyl-Lithium, und außerdem Alkalimetallalkoholate, wie Natriummethylat, Natriumethylat und Kalium-tert.-butylat.

Als Verdünnungsmittel kommen bei der Durchführung des erfindungsgemäßen Verfahrens - (b) alle üblichen inerten organischen Solventien in Betracht. Vorzugsweise verwendbar sind Ether, wie Diethylether, Tetrahydrofuran und Dioxan, und außerdem stark polare Solventien, wie Dimethylformamid und Dimethylsulfoxid.

Die Reaktionstemperaturen können bei der Durchführung des erfindungsgemäßen Verfahrens - (b) innerhalb eines größeren Bereiches variiert werden. Im allgemeinen arbeitet man bei der Behandlung der Verbindungen der Formel (IV) mit starken Basen bei Temperaturen zwischen 0 und 50°C, vorzugsweise zwischen 20 und 30°C. Bei der anschließenden Umsetzung mit Oxiranen der Formel (II) arbeitet man im allgemeinen bei Temperaturen zwischen 20 und 180°C, vorzugsweise zwischen 60 und 150°C.

Das erfindungsgemäße Verfahren (b) wird im allgemeinen unter Normaldruck durchgeführt. Es ist jedoch auch möglich, unter erhöhtem oder vermindertem Druck zu arbeiten.

Bei der Durchführung des erfindungsgemäßen Verfahrens (b) setzt man Acetanilide der Formel - (IV) im allgemeinen mit einer äquivalenten Menge an starker Base um und fügt anschließend eine äquivalente Menge an Oxiran der Formel (II) hinzu. Es ist aber auch möglich, die eine oder andere Komponente in einem Überschuß zu verwenden. Die Aufarbeitung erfolgt nach üblichen Methoden.

Zur Herstellung von Säureadditions-Salzen der Verbindungen der Formel (I) kommen alle diejenigen Säuren in Frage, die zu physiologisch verträglichen Salzen führen. Vorzugsweise verwendbar sind diejenigen Säuren, die bereits im Zusammenhang mit der Beschreibung der erfindungsgemäßen Stoffe als bevorzugt zu addierende Säuren genannt wurden.

Die Säureadditions-Salze der Verbindungen der Formel (I) lassen sich in einfacher Weise nach üblichen Salzbildungsmethoden herstellen. Im allgemeinen verfährt man so, daß man eine Verbindung der Formel (I) in einem geeigneten inerten Verdünnungsmittel löst und dann eine Säure hinzufügt. Die Isolierung erfolgt in bekannter Weise, z.B. dadurch, daß man das Salz abfiltriert und gegebenenfalls durch Waschen mit einem inerten organischen Lösungsmittel reinigt.

Zur Herstellung von Metallsalz-Komplexen der Verbindungen der Formel (I) kommen vorzugsweise Salze von denjenigen Metallen in Betracht, die bereits im Zusammenhang mit der Beschreibung der erfindungsgemäßen Stoffe als bevorzugt zu addierende Metalle genannt wurden. Als Anionen dieser Metallsalze kommen vorzugsweise Halogenwasserstoffsäuren, wie z.B. Chlorwasserstoffsäure und Bromwasserstoffsäure, ferner Phosphorsäure, Salpetersäure und Schwefelsäure in Frage.

Die Metallsalz-Komplexe von Verbindungen der Formel (I) lassen sich in einfacher Weise nach üblichen Methoden herstellen. Im allgemeinen geht man so vor, daß man ein Metallsalz in Alkohol, wie z.B. Ethanol, löst und dann eine Verbindung der Formel (I) hinzufügt. Die Isolierung erfolgt ebenfalls in bekannter Weise, z.B. dadurch, daß man den Metallsalz-Komplex abfiltriert und gegebenenfalls durch Umkristallisation reinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen; so zur Bekämpfung von Erysiphe-Arten, wie z.B. gegen den Erreger des Gersten bzw. des Getreidemehltaus (Erysiphe graminis). Ferner sind die erfindungsgemäßen Wirkstoffe auch sehr gut geeignet zur Bekämpfung der Erreger von Pyricularia und Pellicularia an Reis sowie gegen Rost - (Uromyces appendiculatus) an Bohnen.

Besonders hervorzuheben ist, daß die erfindungsgemäßen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanzen zuführt.

Außerdem besitzen die erfindungsgemäßen Wirkstoffe auch pflanzenwuchsregulierende Eigenschaften.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowei ULV-Formulierungen.

Diese Formulierungen werden in bekannter Weise hergestellt, z.B. duch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder - schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen in wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußchalen, Maiskolben and

Tabakstengel. Als Emulgier-und/oder Schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalo cyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 uns 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %. am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Herstellung und Verwendung der erfindungsgemäßen Wirkstoffe gehen aus den folgenden Beispielen hervor.

Herstellungsbeispiele

Beispiel 1

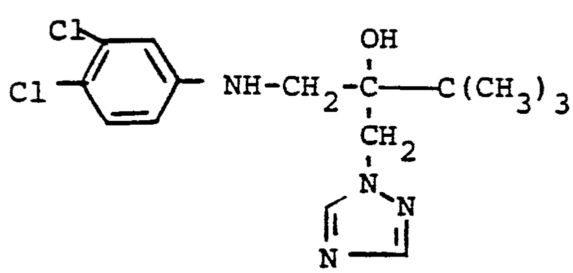

(1)

Ein Gemisch aus 16,2 g (0,1 Mol) 3,4-Dichloranilin und 18,1 g (0,1 Mol) 2-tert.-Butyl-2-(1,2,4-triazol-1-yl-methyl)-oxiran in 150 ml Ethanol und 100 ml Wasser wird 16 Stunden unter RÜckfluß gekocht. Anschließend wird auf Raumtemperatur abgekühlt und durch Abziehen des Lösungsmittels unter vermindertem Druck eingeengt. Der verbleibende Rückstand wird mit Wasser versetzt, und

das entstandene Gemisch wird mit Essigester extrahiert. Die vereinigten organischen Phasen werden unter vermindertem Druck eingeengt, und der verbleibende Rückstand wird chromatographisch gereinigt (Kieselgel-Säule; Laufmittel : Dichlormethan/Essigester = 3:1).

Nach dem Eindampfen des Eluates erhält man 6,7 g (19,5 % der Theorie) an 1-(3,4-Dichlorphenylamino)-3,3-dimethyl-2-(1,2,4-triazol-1-yl-methyl)-butan-2-ol, das nach Umkristalliation aus Acetonitril einen Schmelzpunkt von Fp = 132-133°C aufweist.

Nach der im Beispiel 1 angegebenen Methode sowie gemäß den angegebenen Verfahren werden auch die in der folgenden Tabelle 1 formelmäßig aufgeführten Stoffe erhalten:

Tabelle 1

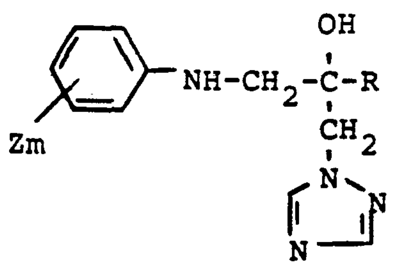

(I)

| Beispiel Nr. | $Z_m$ | R | Schmelzpunkt (°C) |
|---|---|---|---|
| 2 | 4-Cl | $-C(CH_3)_3$ | 87 - 89 |
| 3 | 2,4-Cl | $-C(CH_3)_3$ | 105 - 106 |
| 4 | 3-Cl | $-C(CH_3)_3$ | 110 - 112 |
| 5 | 2,4-$CH_3$ | $-C(CH_3)_3$ | 104 - 105 |

In den folgenden Verwendungsbeispielen wurden die nachstehend angegebenen Verbindungen als Vergleichssubstanzen eingesetzt:

(A) = 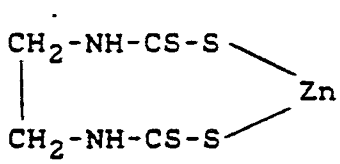

(bekannt aus Phytopathology 33, 1113 (1963)).

(B) = 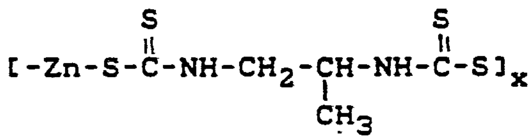

(bekannt)

Beispiel A

Erysiphe-Test (Gerste) / protektiv /

Lösungsmittel: 100 Gewichtsteile Dimethylformamid

Emulgator: 0,25 Gewichsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigen die erfindungsgemäßen Verbindungen (2) und (4) eine bessere Wirksamkeit als die Vergleichssubstanz (A).

Beispiel B

Erysiphe-Test (Gerste) / kurativ /

Lösungsmittel: 100 Gewichtsteile Dimethylformamid Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung taufeucht besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

In diesem Test zeigt die erfindungsgemäße Verbindung (2) eine bessere Wirksamkeit als die Vergleichssubstanz (A).

Beispiel C

Uromyces-Test (Buschbohne) / protektiv /

Lösungsmittel: 4,7 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22°C und 100 % relativer Luftfeuchtigkeit.

Die Pflanzen werden dann unter intensiver Belichtung für 9 Tage bei 20 bis 22°C und einer relativen Luftfeuchtigkeit von 70 bis 80 % im Gewächshaus aufgestellt.

10 Tage nach der Inokulation erfolgte die Auswertung.

In diesem Test zeigt die erfindungsgemäße Verbindung (4) eine bessere Wirksamkeit als die Vergleichssubstanz (B).

Beispiel D

Pyricularia-Test (Reis) /protektiv /

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

In diesem Test zeigen die erfindungsgemäßen Verbindungen (2) und (4) eine bessere Wirkung als die Vergleichssubstanz (A).

Beispiel E

Pyricularia-Test (Reis) / kurativ /

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf kurative Wirksamkeit werden junge Reispflanzen mit einer wäßrigen Sporensuspension von Pyricularia oryzae inokuliert. Die Pflanzen bleiben in einem Gewächshaus 16 Std. bei 25°C und einer rel. Luftfeuchtigkeit von 100 % stehen. Nach einer kurzen Abtrocknungszeit werden die Pflanzen mit der Wirkstoffzubereitung tropfnaß gespritzt.

4 Tage nach der Inokulation erfolgt die Auswrtung des Krankheitsbefalls.

In diesem Test zeigt die erfindungsgemäße Verbindung (4) eine bessere Wirksamkeit als die Vergleichssubstanz (A).

Beispiel F

Pyricularia-Test (Reis) / systemisch /

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

In diesem Test zeigen die erfindungsgemäßen Verbindungen (2) und (4) eine bessere Wirksamkeit als die Vergleichssubstanz (A).

Beispiel G

Pellicularia-Test (Reis)

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3-bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

In diesem Test zeigen die erfindungsgemäßen Verbindungen (2) und (4) eine bessere Wirksamkeit als die Vergleichssubstanz (A).

## Ansprüche

1. Triazol-Derivate der Formel

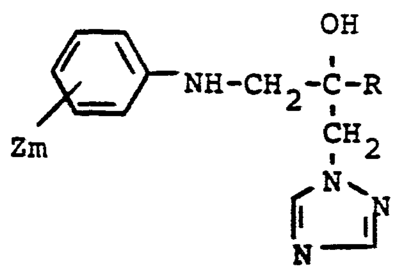

(I)

in welcher

R für Alkyl, Cycloalkyl oder für die Gruppierungen

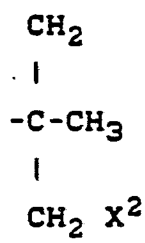

oder

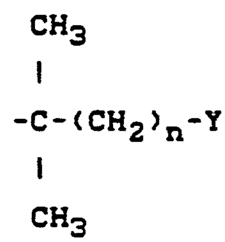

steht,

worin

$X^1$ für Wasserstoff oder Halogen steht,

$X^2$ für Halogen steht,

Y für Alkoxy, Alkylthio, Halogenalkoxy, Halogenalkylthio, Alkenyl, Alkoxcarbonyl, Cyano, gegegebenenfalls substituiertes Phenyl, gegebenenfalls substituiertes Phenoxy, gegebenenfalls substituiertes Phenylthio, gegebenenfalls substituiertes Phenylalkoxy oder gegebenenfalls substituiertes Phenylalkylthio steht und n für die Zahlen 0, 1 oder 2 steht,

Z für Halogen, Alkyl, Halogenalkyl, Halogenalkoxy, Halogenalkylthio oder Phenyl steht und

m für die Zahlen 0, 1, 2 oder 3 steht,

sowie deren Säureadditions-Salze und Metallsalz-Komplexe.

2. Triazol-Derivate der Formel (I). in denen

R für geradkettiges oder verzweigtes Alkyl mit 1 bis 6 Kohlenstoffatomen, Cycloakiyl mit 3 bis 8 Kohlenstoffatomen oder für die Gruppierungen der Formeln

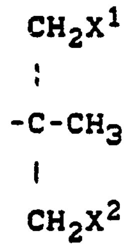

und

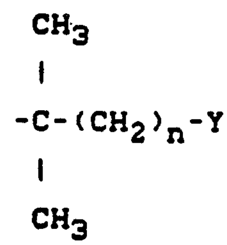

steht,

worin

$X^1$ für Wasserstoff, Fluor, Chlor oder Brom steht,

$X^2$ für Fluor, Chlor oder Brom steht,

Y für Alkoxy mit 1 bis 6 Kohlenstoffatomen, Alkylthio mit 1 bis 6 Kohlenstoffatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Alkenyl mit 2 bis 6 Kohlenstoffatomen, Alkoxycarbonyl mit 1 bis 4 Kohlenstoffatomen im Alkoxyteil, Cyano, Phenyl, Phenoxy, Phenylalkoxy mit 1 bis 4Kohlenstoffatomen im Alkoxyteil und Phenylalkylthio mit 1 bis 4 Kohlenstoffatomen im Alkylthioteil steht, wobei jeder dieser Phenyl-, Phenoxy-, Phenylakoxy-und Phenylalkylthio-Reste ein-oder mehrfach, gleichartig oder verschieden substituiert sein kann durch Halogen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cyclohexyl, Dialkylamino mit 1 bis 4 Kohlenstoffatomen in jedem Alkylteil, Nitro, Cyano und Alkoxycarbonyl mit 1 bis 4 Kohlenstoffatomen im Alkoxyteil,

und

n für die Zahlen 0, 1 oder 2 steht,

Z für Fluor, Chlor, Brom, Alkyl, mit 1 bis 4 Kohlenstoffatomen, Halogenalkyl mit 1 oder 2 Kohlenstoffatomen und 1 bis 5 gleichen oder verschiedenen Halogenatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen oder für Phenyl steht und m für die Zahlen 0, 1, 2 oder 3 steht.

3. Verfahren zur Herstellung von Triazol-Derivaten der Formel

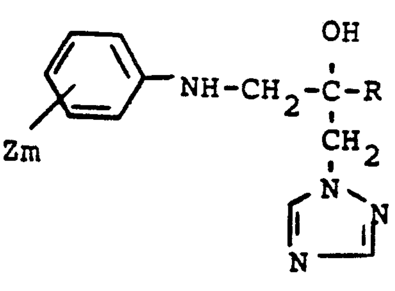

(I)

in welcher

R für Alkyl, Cycloalkyl oder für die Gruppierungen

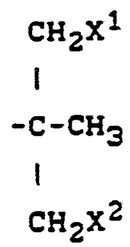

oder

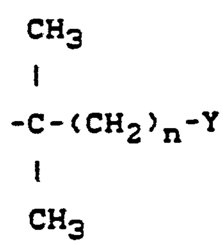

steht,

worin

$X^1$ für Wasserstoff oder Halogen steht,

$X^2$ für Halogen steht,

Y für Alkoxy, Alkylthio, Halogenalkoxy, Halogenalkylthio, Alkenyl, Alkoxycarbonyl, Cyano, gegebenenfalls substituiertes Phenyl, gegebenenfalls substituiertes Phenoxy, gegebenenfalls substituiertes Phenylthio, gegebenenfalls substituiertes Phenylalkoxy oder gegebenenfalls substituiertes Phenylalkylthio steht und

n für die Zahlen 0, 1 oder 2 steht,

Z für Halogen, Alkyl, Halogenalkyl, Halogenalkoxy, Halogenalkylthio oder Phenyl steht und

m für die Zahlen 0, 1, 2 oder 3 steht,

sowie deren Säureadditions-Salzen und Metallsalz-Komplexen, dadurch gekennzeichnet, daß man

a) Oxirane der Formel

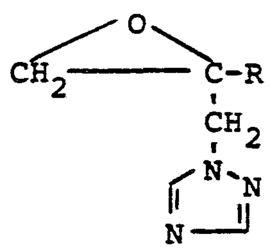

(II)

in welcher

R die oben angegebene Bedeutung hat,

mit Anilin-Derivaten der Formel

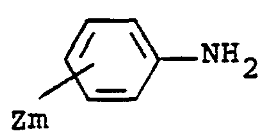

(III)

in welcher

Z und m die oben angegebene Bedeutung haben,

in Gegenwart eines Verdünnungsmittels umsetzt;

oder

b) Acetanilide der Formel

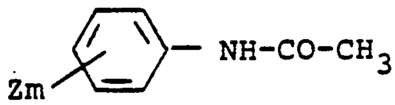

(IV)

in welcher

Z und m die oben angegebene Bedeutung haben,

mit starken Basen in Gegenwart eines Verdünnungsmittels behandelt und die dabei entstehenden Produkte anschließend mit Oxiranen der Formel

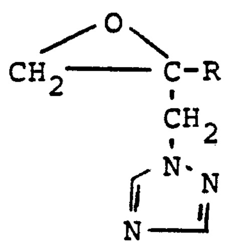

(II)

in welcher

R die oben angegebene Bedeutung hat,

in Gegenwart eines Verdünnungsmittels umsetzt;

und gegebenenfalls anschließend an die erhaltenen Verbindungen der Formel (I) eine Säure oder ein Metallsalz addiert.

4. Fungizide und pflanzenwachstumsregulierende Mittel, gekennzeichnet durch einen Gehalt an mindestens einem Triazol-Derivat der Formel (I) bzw. einem Säureadditions-Salz oder Metallsalz-Komplex eines Triazol-Derivates der Formel (I).
5. Verfahren zur Bekämpfung von Pilzen sowie zur Regulierung des Pflanzenwachstums, dadurch gekennzeichnet, daß man Triazol-Derivate der Formel (I) bzw. deren Säureadditions-Salze oder Metallsalzkomplexe auf die Pflanzen und/oder deren Lebensraum bzw. auf die Pilze und/oder deren Lebensraum ausbringt.
6. Verwendung von Triazol-Derivaten der Formel (I) bzw. von deren Säureadditions-Salzen oder Metallsalz-Komplexen zur Bekämpfung von Pilzen bzw. zur Regulierung des Pflanzenwachstums.
7. Verfahren zur Herstellung von fungiziden und pflanzenwachstumsregulierenden Mitteln, dadurch gekennzeichnet, daß man Triazol-Derivate der Formel (I) bzw. deren Säureadditions-Salze oder Metallsalz-Komplexe mit Streckmitteln und/oder oberflächenaktiven Stoffen vermischt.